# EUROPEAN PATENT APPLICATION

(11) **EP 2 000 163 A2**
(43) Date of publication of application: **10.12.2008**
(21) Application number: 07714846.8
(22) Date of filing: 23.02.2007
(51) Int. Cl.: A61M 5/178, A61M 5/28

(54) **SYRINGE CYLINDER**

(30) Priority: 28.02.2006 JP 2006053976
(71) Applicant: Hisamitsu Pharmaceutical Co., Inc., Tosu-shi, Saga 841-0017 (JP)
(72) Inventor: MUTA, Kazunori, Tosu-shi, Saga 8410017 (JP); OTA, Shigeo, Chiyoda-ku, Tokyo 1006221 (JP); YOSHINAGA, Takaaki, Tosu-shi, Saga 8410017 (JP); SHIGIISHI, Masakazu, Chiyoda-ku, Tokyo 1006221 (JP); KUBO, Junichi, Tosu-shi, Saga 8410017 (JP); TSUTSUMI, Nobuo, Chiyoda-ku, Tokyo 1006221 (JP)
(74) Representative: Grünecker, Kinkeldey, Stockmair & Schwanhäusser Anwaltssozietät
(86) International application number: PCT/JP2007/053375
(87) International publication number: WO 2007/099870

(57) **Abstract**

A syringe cylinder 12 includes a groove 32 formed in an inner peripheral surface thereof and extending over a predetermined length from an opening end 30 thereof. Even with the intermediate sliding stopper 14 located at a position where the groove 32 is formed, the pressure inside the syringe cylinder 12 can be reduced. Thus, during vacuum tapping, the intermediate sliding stopper 14 can be held in the vicinity of the opening end 30 of the syringe cylinder 12. Consequently, the intermediate sliding stopper 14 can be inserted into the syringe cylinder 12 in a correct posture and reliably moved to a predetermined position in the syringe cylinder 12.

## Description

### Technical Field

The present invention relates to a syringe cylinder suitable for a kit drug formulation, and in particular, to a syringe cylinder suitable for a kit drug formulation and which allows two types of liquids to be sequentially injected.

### Background Art

Kit drug formulations are products including syringe cylinders pre-filled with drugs. With a kit drug formulation called a series sequential dispensation type, two types of drugs can be sequentially injected by a single injection operation without being mixed together. Various types of series sequential dispensation kit drug formulations are available. However, in general, an intermediate sliding stopper is provided in the syringe cylinder, with different types of drugs filled in a front space and a rear space in the syringe cylinder which are partitioned by the intermediate sliding stopper. As a plunger is pushed in, the intermediate sliding stopper slides through the syringe cylinder toward a syringe port. The drug located on the syringe port side (located forward) first flows out. When the intermediate sliding stopper reaches a syringe port-side end of the syringe cylinder, the sealing condition established by the intermediate sliding stopper is canceled to allow the rear drug to flow out through the syringe port.

Some intermediate sliding stoppers are simply cylindrical. Such an intermediate sliding stopper is subjected to significant friction in the syringe cylinder and thus has disadvantages such as an improper sliding operation. Thus, to allow the intermediate sliding stopper to slide smoothly and to prevent the rear drug from flowing into the front drug during injection, for example, a conventional kit drug formulation such as the one described in Patent Document 1 has been proposed. In the kit drug formulation described in Patent Document 1, an intermediate sliding stopper is composed of a cylindrical main body portion with an outer diameter smaller than the inner diameter of a syringe cylinder, and an annular lip portion protrusively provided on one end side (front end side) of an outer peripheral surface of the main body portion. The outer diameter of the lip portion is slightly larger than the inner diameter of the syringe cylinder. Thus, the spaces located in front of and behind the intermediate sliding stopper can be partitioned from each other in a liquid tight manner. Furthermore, the contact area between an inner peripheral surface of the syringe cylinder and the lip portion is small. The syringe cylinder and the lip portion are thus almost in a line contact condition to allow the intermediate sliding stopper to slide smoothly.

In the kit drug formulation described in Patent Document 1, three or four pairs of projections are formed on the inner peripheral surface of the syringe cylinder and adjacent to a syringe port. When the lip portion of the intermediate sliding stopper reaches the position of the pairs of projections, the pairs of projections compressively deform the lip portion to form, between the projections of each pair, a bypass passage via which spaces located in front of and behind the intermediate sliding stopper communicate with each other. Thus, the rear drug is guided to the syringe port.

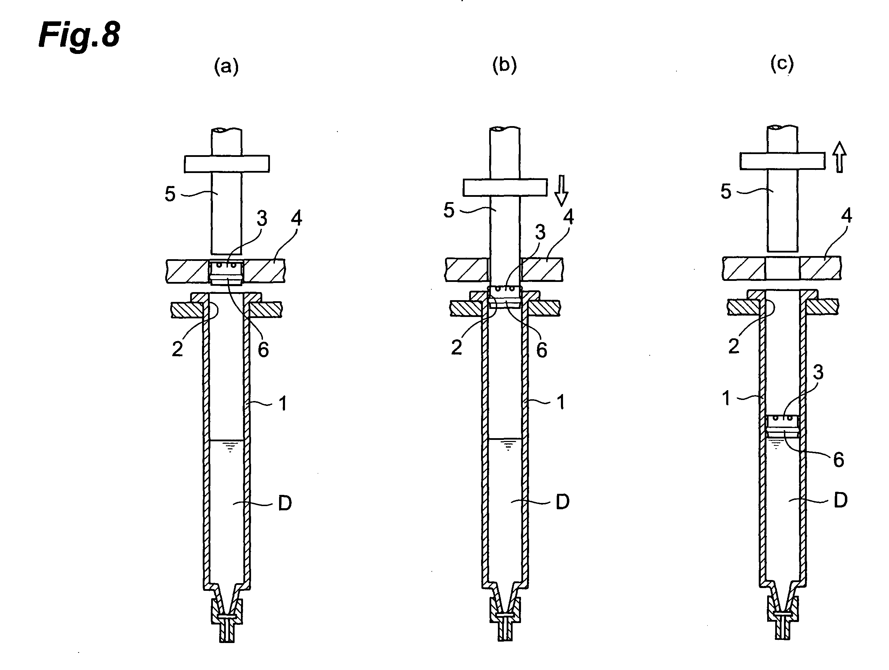

With a kit drug formulation such as the one described above, if the front drug is filled into the syringe cylinder and the intermediate sliding stopper is then placed in the syringe cylinder, then for example, a technique called vacuum tapping such as the one described in, for example, Patent Document 2 described below is used. The vacuum tapping method will be described with reference to Figure 8.

First, the syringe cylinder 1 is placed in a vacuum box (not shown in the drawings) in a vertical condition with an opening end 2 thereof faced upward. A drug D is introduced into the syringe cylinder 1. Furthermore, an intermediate sliding stopper 3 is held above the opening end 2 of the syringe cylinder 1 by a holder 4 (Figure 8(a)). In this condition, the air in the vacuum box is discharged to the exterior by an appropriate vacuum pump. When the pressure in the vacuum box decreases to a predetermined value, a stoppering bar 5 located above the holder 4 is lowered to push the intermediate sliding stopper 3 into the opening end 2 of the syringe cylinder 1 (Figure 8(b)). At least after a lip portion 6 of the intermediate sliding stopper 3 comes into contact with an inner peripheral surface of the syringe cylinder 1, the pressure in the vacuum box is returned to a ordinary pressure. Then, since the space between the intermediate sliding stopper 3 and the drug D in the syringe cylinder 1 is vacuum, even when the stoppering bar 5 is pulled up, the intermediate sliding stopper 3 lowers through the syringe cylinder 1. Thus, the tip of the intermediate sliding stopper 3 comes into contact with a surface of the drug D (Figure 8(c)).
Patent Document 1: Japanese Patent Laid-Open No. 2005-131216
Patent Document 2: Japanese Patent Publication No. 4-28386

However, if a vacuum tapping method such as the one described above is used to place the intermediate sliding stopper in the syringe cylinder of the conventional series sequential dispensation kit drug formulation, various problems may result from the circular cross section of the inner peripheral surface of the opening end of the syringe cylinder.

For example, if the intermediate sliding stopper is cylindrical, since the inner diameter of the opening end of the syringe cylinder is almost the same as the outer diameter of the intermediate sliding stopper, a sufficient distance needs to be set between the intermediate sliding stopper and the opening end of the syringe cylinder in the condition in Figure 8(a) in order to efficiently reduce the pressure in the space in the syringe cylinder. As a result, if the internal components of the vacuum box and the syringe cylinder are inaccurately positioned, when the stoppering bar is used to push the intermediate sliding stopper into the syringe cylinder, the intermediate sliding stopper may come into contact with an edge of the opening end of the syringe cylinder. The intermediate sliding stopper may thus be prevented from being inserted into the syringe cylinder.

On the other hand, for the intermediate sliding stopper described in Patent Document 1, the outer diameter of a lower end (a part located below the lip portion) of the intermediate sliding stopper is smaller than the inner diameter of the opening end of the syringe cylinder. The lower end of the intermediate sliding stopper can thus be reliably inserted into the opening end of the syringe cylinder. However, since the lip portion comes into line contact with the inner peripheral surface of the syringe cylinder, the axis of the intermediate sliding stopper may be tilted to the axis of the syringe cylinder immediately after the insertion. In this condition, a gap may be created between the intermediate sliding stopper and the inner peripheral surface of the syringe cylinder. Then, the intermediate sliding stopper cannot be lowered to a predetermined position.

### Disclosure of the Invention

### Problem to be solved by the Invention

An object of the present invention is to provide a syringe cylinder that allows the above-described problems to be solved.

To accomplish this object, a syringe cylinder according to the present invention includes a groove formed in an inner peripheral surface thereof so as to extend over a predetermined distance from an opening end thereof. In particular, the syringe cylinder is suitable for a series sequential dispensation kit drug formulation including an intermediate sliding stopper located inside.

Even with the intermediate sliding stopper located at the position where the groove is formed, the pressure inside the syringe cylinder can be reduced. Thus, during vacuum tapping, the intermediate sliding stopper can be held in the vicinity of the opening end of the syringe cylinder. Furthermore, the intermediate sliding stopper can be pre-inserted into the opening end of the syringe cylinder so as to enable a reduction in the pressure in the syringe cylinder. Thus, the intermediate sliding stopper can be placed in the syringe cylinder in a correct posture and reliably moved to a predetermined position in the syringe cylinder.

A predetermined length of the groove may be equal to smaller than a length of the intermediate sliding stopper.

Furthermore, at least a pair of projections may be formed on an inner peripheral surface of a syringe port side so as to deform the intermediate sliding stopper to form, between the projections of the pair, a bypass passage via which areas located in front of and behind the intermediate sliding stopper communicate with each other.

Moreover, to stop the intermediate sliding stopper at a desired position in the syringe cylinder during vacuum tapping, the inner peripheral surface of the syringe cylinder is tapered such that a diameter decreases from the opening end toward the syringe port.

### Effect of the Invention

As described above, the use of the syringe cylinder according to the present invention enables the intermediate sliding stopper to be easily and reliably mounted, allowing a kit drug formulation to be more efficiently manufactured.

### Brief Description of the Drawings

Figure 1 is a perspective view showing a kit drug formulation including a syringe cylinder according to the present invention;
Figure 2 is a vertical sectional view of the kit drug formulation in Figure 1;
Figure 3 is a vertical sectional view of a syringe cylinder according to the present invention;
Figure 4 is a sectional view of the syringe cylinder taken along line IV-IV in Figure 3;
Figure 5 is a sectional view of the syringe cylinder taken along line V-V in Figure 2, the view showing that an intermediate sliding stopper has reached the tip of the syringe cylinder;
Figure 6 is a diagram showing a procedure ((a) to (c)) of a vacuum tapping operation of placing an intermediate sliding stopper in a syringe cylinder according to the present invention;
Figure 7 is a sectional view of the syringe cylinder taken along line VII-VII in Figure 6; and
Figure 8 is a diagram showing a procedure ((a) to (c)) of a vacuum tapping operation of placing an intermediate sliding stopper in a conventional syringe cylinder.

### Description of Symbols

10 ... kit drug formulation, 12 ... syringe cylinder, 14 ... intermediate sliding stopper, 16 ... main body portion, 18 ... syringe port, 20 ... lip portion, 22 ... plunger, 24 ... protrusion, 26 ... projection, 28 ... syringe needle, 30 ... opening end, 32 ... groove, 34 ... raised portion, 50 ... holder, 52 ... stoppering bar.

### Best Modes for Carrying Out the Invention

A preferred embodiment of the present invention will be described below in detail with reference to the drawings.

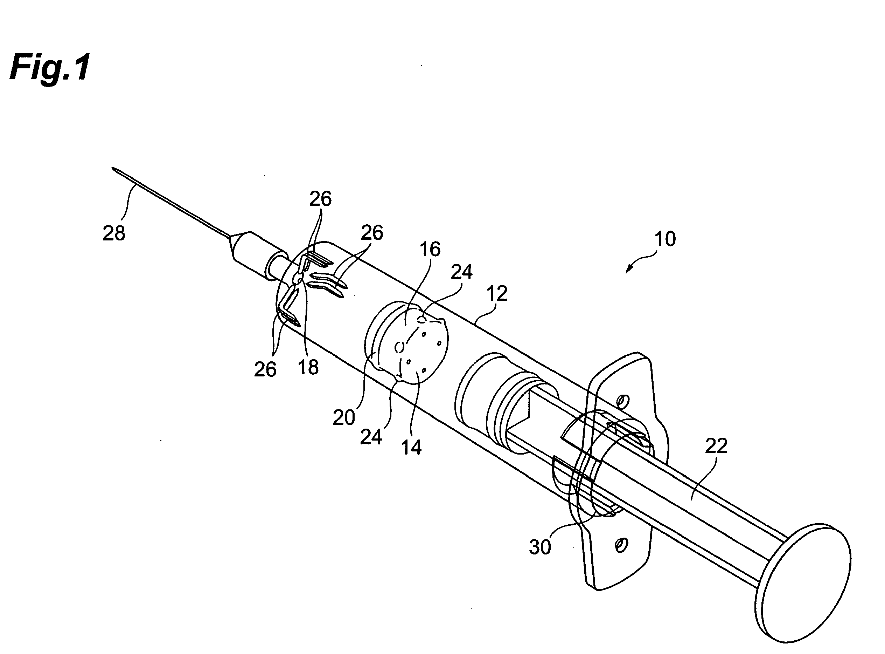

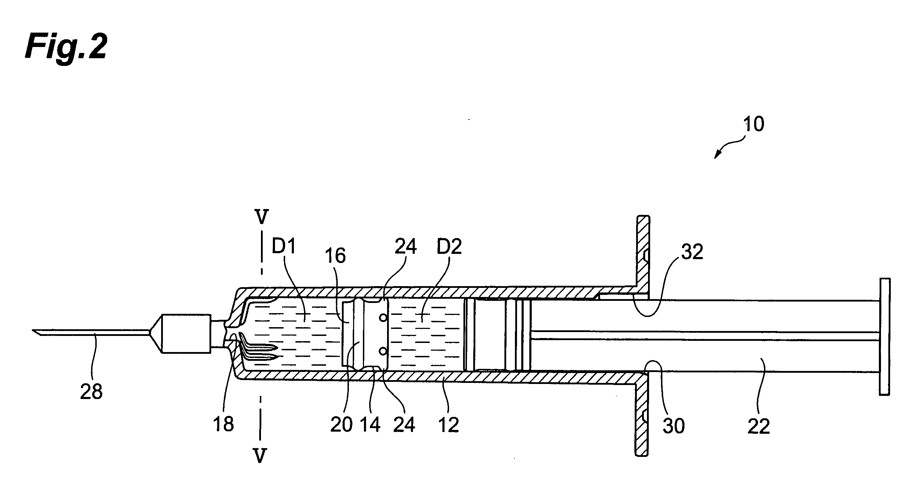

Figures 1 and 2 show a series sequential dispensation kit drug formulation 10 made up of a syringe cylinder according to the present invention. The illustrated kit drug formulation 10 is basically similar to the above-described conventional configuration. The kit drug formulation 10 includes an intermediate sliding stopper 14 inside a syringe cylinder 12. The intermediate sliding stopper 14 partitions the interior of the syringe cylinder 12 into two spaces in which different types of drugs D1 and D2 are filled.

The intermediate sliding stopper 14 is an integrally formed component obtained by subjecting surfaces of a rubber elastomer to silicon coating or Teflon (registered trademark) processing, for example. The intermediate sliding stopper 14 is composed of a substantially cylindrical main body portion 16, an annular lip portion 20 formed on a front end side (syringe port 18 side) of an outer peripheral surface of the main body portion 16, and a plurality of (in the illustrated embodiment, six) protrusions 24 formed on a rear end side (plunger 22 side) of the outer peripheral surface of the main body portion 16. The outer diameter of the lip portion 20 is slightly larger than the inner diameter of the syringe cylinder 12. The lip portion 20 partitions a front space and a rear space in the syringe cylinder 12 from each other in a liquid tight manner. Furthermore, the distance between a central point of the main body portion 16 and a top of each of the protrusions 24 is equivalent to or slightly larger than the half of the inner diameter of the syringe cylinder 12. Therefore, a rear portion of the main body portion 16 is supported by the protrusions 24. Thus, the intermediate sliding stopper 14 is maintained coaxially with the syringe cylinder 12 by the protrusions 24 and the lip portion 20. This prevents, for example, the following situation: the intermediate sliding stopper 14 slides tiltingly to mix the front and rear drugs D1 and D2 together across the intermediate sliding stopper 14.

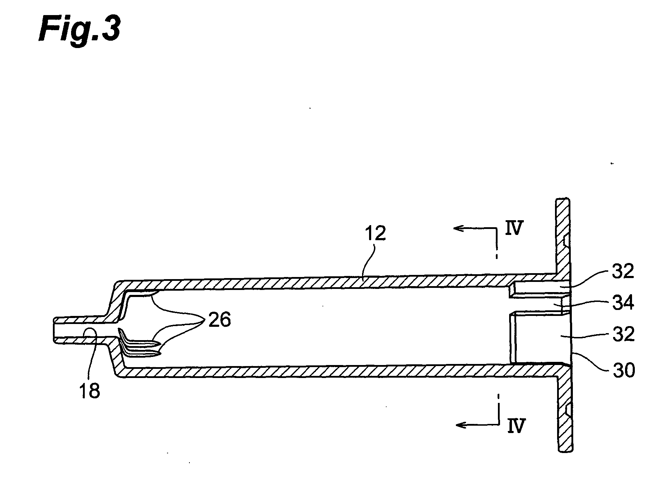

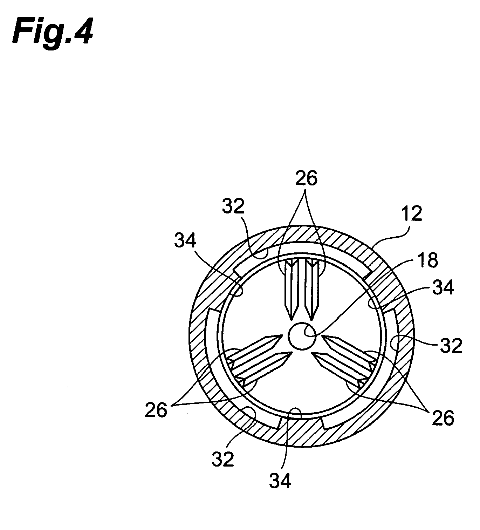

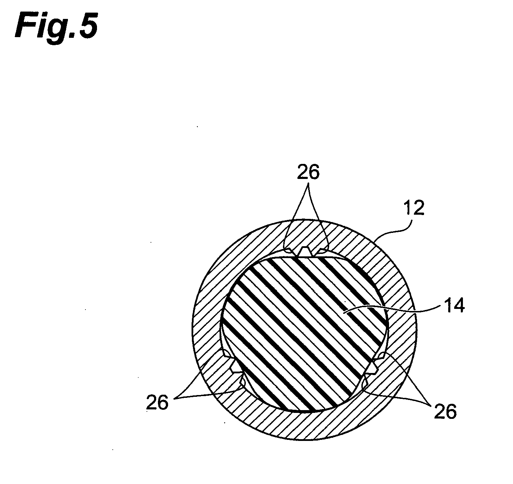

As is also appreciated from Figures 3 and 4, plural pairs of projections 26 are formed on an inner peripheral surface of a tip side (syringe port 18 side) of the syringe cylinder 12. The length of each of the projections 26 on an inner peripheral surface of the syringe cylinder 12 is smaller than the entire length of the intermediate sliding stopper 14 but is larger than the length from the front end to the lip portion 20. Furthermore, the projection 26 extends to an inner surface of the tip surface of the syringe cylinder 12. When the plunger 22 is pushed into the syringe cylinder 12, the front drug D1 flows out first through the syringe port 18 and then through the syringe needle 28. Furthermore, the intermediate sliding stopper 14 slides forward and reaches the tip of the syringe cylinder 12. In this position, as shown in Figure 5, the projections 26 dent the lip portion 20 of the intermediate sliding stopper 14. The gap between the projections 26 of each pair forms a bypass channel along which the rear drug D2 is guided to the syringe port 18. Thus, after injection of the front drug D1 is completed, the rear drug D2 can be successively injected.

The distance between the projections 26 of each pair can be appropriately set so as to provide the bypass channel. However, provided that the rear drug D2 is viscous, a relatively long distance is preferably set between the projections 26. Furthermore, the number of pairs of projections 26 is three in the illustrated embodiment, but is not limited. At least one pair has only to be provided, and four or more pairs may be provided if the speed at which the rear drug D2 is injected needs to be increased.

As is appreciated from Figure 4, a plurality of arc grooves 32 are formed at equal intervals in a circumferential direction in the inner peripheral surface of the syringe cylinder 12 on an opening end 30 side thereof. The grooves 32 shape the area 34 between the grooves 32 like an arc raised portion. In the illustrated embodiment, the arc length of each of the grooves 32 is longer than that of the raised portion 34. Furthermore, the length of the groove 32 (the length along the axis of the syringe cylinder 12) is equivalent to or shorter than (preferably slightly shorter than) that of the intermediate sliding stopper 14. That is, the groove 32 extends through the inner peripheral surface of the syringe cylinder 12 over a predetermined length from the opening end 30. The predetermined length in the axial direction is equivalent to or shorter than the length of the intermediate sliding stopper. The diameter of the inner peripheral edge of the opening end 30 increases outward in order to further facilitate the insertion of the intermediate sliding stopper 14.

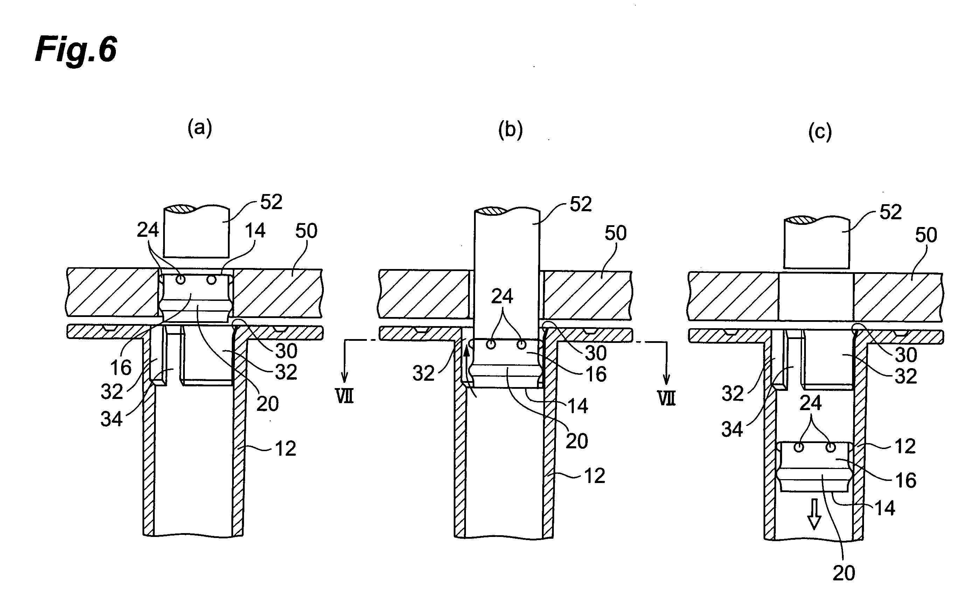

Now, with reference to Figure 6, the case will be described where after the front drug D1 is filled into the syringe cylinder 12 configured as described above, the intermediate sliding stopper 14 is placed in the syringe cylinder 12 by the vacuum tapping method.

First, the syringe cylinder 12 with a cap (not shown in the drawings) attached to the syringe port 18 and with the drugs filled therein is placed in a vacuum box (not shown in the drawings) in a vertical condition with the opening end 30 faced upward. Then, as shown in Figure 6(a), the intermediate sliding stopper 14 is held by the holder 50 with the lip portion 20 located lower side. The intermediate sliding stopper 14 is placed above the opening end 30 of the syringe cylinder 12. The distance between the intermediate sliding stopper 14 and the opening end 30 of the syringe cylinder 12 can be effectively minimized. Furthermore, although not shown in the drawings, the intermediate sliding stopper 14 may be held with the lower end of the intermediate sliding stopper 14 placed in the opening end 30 of the syringe cylinder 12. That is, the vacuum tapping method requires a reduction in the pressure inside the syringe cylinder 12. However, since the grooves 32 are formed in the opening end 30-side inner peripheral surface of the syringe cylinder 12, provided that the intermediate sliding stopper 14 is located above the position of a lower end of each of the grooves 32, the grooves 32 serves as outflow paths for air. This enables a reduction in the pressure in the syringe cylinder 12.

Then, the air in the vacuum box is discharged by a vacuum pump to start pressure reduction. Then, the intermediate sliding stopper 14 is tapped. The intermediate sliding stopper 14 is tapped by lowering the stoppering bar 52. As shown in Figure 6(b), the intermediate sliding stopper 14 is placed in an area of the syringe cylinder 12 in which the grooves 32 are formed. In the tapping step, the intermediate sliding stopper 14 is held in the vicinity of the opening end 30 of the syringe cylinder 12 as described above. Thus, the lower end of the intermediate sliding stopper 14 can be reliably inserted into the syringe cylinder 12. Furthermore, even when the lip portion 20 of the intermediate sliding stopper 14 advances sufficiently into the syringe cylinder 12, the intermediate sliding stopper 14 is prevented from entering the syringe cylinder 12 obliquely since the protrusions 24 are held by the holder 50.

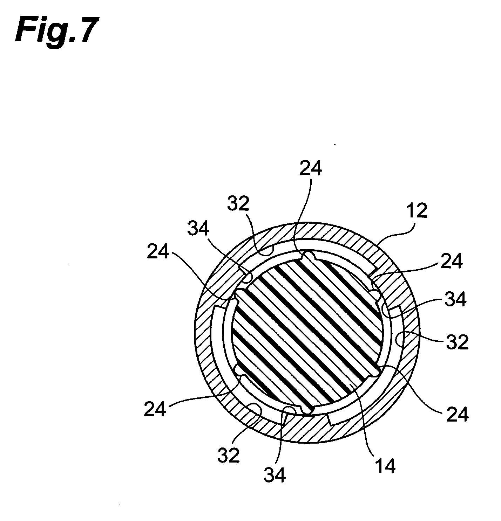

In the prior art, the tapping of the intermediate sliding stopper 14 is not performed until the pressure in the vacuum box reaches a predetermined value. However, when the syringe cylinder 12 according to the present invention is used, tapping may be performed simultaneously with or before the start of pressure reduction. In the position shown in Figure 6(b), as is also appreciated from Figure 7, the intermediate sliding stopper 14 is supported by the raised portion 34. A part of the internal space of the syringe cylinder 12 located below the intermediate sliding stopper 14 communicates with the exterior through the grooves 32. This enables a reduction in the pressure in the syringe cylinder 12. Consequently, the intermediate sliding stopper 14 can also be placed on the opening end 30 side of the syringe cylinder 12 before the reduction in the pressure in the vacuum box is started or before the intermediate sliding stopper 14 is placed in the vacuum box. This enables a reduction in the time required for the vacuum tapping operation for the intermediate sliding stopper 14. As a result, the kit drug formulation 10 can be more efficiently manufactured.

Once the pressure in the vacuum box reaches a predetermined value, the intermediate sliding stopper 14 is further pushed into the syringe cylinder 12. Then, after the entire circumference of the lip portion 20 comes into contact with the inner peripheral surface of the syringe cylinder 12, the vacuum box is opened and returned to the ordinary pressure. Then, as shown in Figure 6(c), the vacuum in the syringe cylinder 12 moves the intermediate sliding stopper 14 to a position where the intermediate sliding stopper 14 comes into contact with the surface of the drug. As described above, the axis of the intermediate sliding stopper 14 is substantially parallel with the axis of the syringe cylinder 12. Thus, this moving step is reliably carried out, and the vacuum tapping operation is completed.

To smoothly move and reliably stop the intermediate sliding stopper 14 at the desired position, the inner peripheral surface of the syringe cylinder 12 is preferably tapered such that the diameter of the syringe cylinder 12 decreases very slightly from the opening end 30 toward the syringe port 18 so that significant friction occurs at the desired stop position of the intermediate sliding stopper 14.

The preferred embodiment of the present invention has been described in detail. However, of course, the present invention is not limited to the above-described embodiment. For example, in the above-described embodiment, the intermediate sliding stopper includes the lip portion. However, the syringe cylinder according to the present invention is applicable to an intermediate sliding stopper of a different shape, such as a generally cylindrical intermediate sliding stopper. Furthermore, the syringe cylinder with the grooves formed on the opening end side according to the present invention is also effective in vacuum-tapping an elastic end stopper located at the tip of the plunger. Therefore, the present invention is also applicable to a normal kit drug formulation with no intermediate sliding stopper.

### Industrial Applicability

The present invention can provide a syringe cylinder which is suitable for the series sequential dispensation kit drug formulation and which allows the intermediate sliding stopper to be reliably placed by the vacuum tapping method.

## Claims

1. A syringe cylinder comprising a groove formed in an inner peripheral surface thereof so as to extend over a predetermined length from an opening end thereof.

2. The syringe cylinder according to claim 1, **characterized in that** an intermediate sliding stopper is placed inside the syringe cylinder.

3. The syringe cylinder according to claim 2, **characterized in that** a predetermined length of the groove is equal to or shorter than a length of the intermediate sliding stopper.

4. The syringe cylinder according to claim 2 or 3, **characterized in that** at least one pair of projections is formed on the inner peripheral surface on a syringe port side so as to deform the intermediate sliding stopper to form, between the projections of the pair, a bypass passage via which areas located in front of and behind the intermediate sliding stopper communicate with each other.

5. The syringe cylinder according to any one of claims 1 to 4, **characterized in that** the inner peripheral surface is tapered such that a diameter decreases from the opening end toward the syringe port.
